# FASCICULE DE BREVET EUROPEEN

(11) **EP 1 229 951 B1**
(45) Date de publication et mention de la délivrance du brevet: **21.01.2004**
(21) Numéro de dépôt: 00971480.9
(22) Date de dépôt: 23.10.2000
(51) Int. Cl.: A61M 5/30

(54) **SERINGUE SANS AIGUILLE AVEC UN MOYEN DE POUSSEE TEMPORAIREMENT RETENU**
NADELLOSE SPRITZE MIT ZEITWEISE FESTGELEGTEM BEWEGUNGSMITTEL
NEEDLELESS SYRINGE WITH TEMPORARILY RETAINED THRUSTING MEANS

(30) Priorité: 05.11.1999 FR 9913850
(43) Date de publication de la demande: 14.08.2002
(73) Titulaire: CROSSJECT, 75181 Paris Cedex 04 (FR)
(72) Inventeur: ALEXANDRE, Patrick, F-70100 Gray (FR); BROUQUIERES, Bernard, F-83100 Toulon (FR); GAUTIER, Philippe, F-91220 Le Plessis Pate (FR); ROLLER, Denis, F-91590 La Ferte Alais (FR)
(74) Mandataire: Waligorski, Carol
(86) Numéro de dépôt international: PCT/FR2000/002943
(87) Numéro de publication internationale: WO 2001/032243

(56) Documents cités:
- EP-A- 0 888 790
- WO-A-97/13537
- US-A- 3 802 430
- US-A- 4 124 024

## Description

La présente invention est dans le domaine des seringues sans aiguille utilisées pour les injections intradermiques, sous-cutanées ou intramusculaires de principe actif liquide à usage thérapeutique en médecine humaine ou vétérinaire.

Par définition une seringue sans aiguille est non invasive : il n'y a pas d'aiguille qui traverse la peau pour amener le principe actif là où il doit agir. Pour une seringue sans aiguille, il faut que le jet de principe actif liquide sortant d'un orifice ou conduit d'injection perce la peau et pénètre plus ou moins profondément suivant le type d'injection souhaité : pour cela le jet doit avoir une grande vitesse. Si le jet est trop lent, il n'y a pas perforation de la peau, le liquide se répand à la surface de la peau et il est perdu car il ne produit pas d'effet thérapeutique.

Dans la plupart des seringues les dispositifs pour refouler le principe actif liquide à travers l'injecteur sont en général des parois déplaçables du type piston ou du type membrane déformable qui doivent être déplacées ou déformées rapidement, avec une forte accélération, pour produire rapidement un jet de vitesse élevée pour percer la peau.

Dans le brevet US 2 322 245, LOCKHART décrit des seringues sans aiguille dont la paroi déplaçable est déplacée ou motorisée par soit un ressort mécanique comprimé, soit une réserve de gaz comprimé. Ce dispositif moteur agit directement sur une tige qui va impacter un piston de refoulement situé à une distance relativement importante ; sur cette distance la tige est accélérée par la détente du ressort, elle impacte le piston de refoulement, le met en mouvement et le déplace assez rapidement pour nébuliser, à travers un conduit assez fin, le principe actif et l'injecter.

Par contre pour des seringues à moteur pyrotechnique, décrites dans le même brevet ou les brevets US 3 802 430 et US 4 124 024, la charge pyrotechnique agit soit directement sur le piston de refoulement, soit par l'intermédiaire d'un soufflet en appui sur ledit piston.

Plus récemment la demande de brevet WO 95/03844, sur des seringues sans aiguilles, reprend sensiblement la même technique d'une tige d'impact agissant sur un piston de refoulement. La tige d'impact est motorisée par un ressort mécanique ou une réserve de gaz comprimés. Le dispositif moteur est dans un état d'énergie élevé, il agit directement sur la tige d'impact qui est retenue par un verrou empêchant ainsi le ressort ou le gaz comprimés de se détendre. Pendant tout le stockage avant utilisation, la tige et le verrou sont fortement précontraints.

L'ouverture dudit verrou, directement ou par l'intermédiaire d'une came, libère la tige d'impact et l'énergie stockée qui déplacent les pièces mobiles, injecte le liquide. Le système passe d'un niveau d'énergie élevé à un niveau faible en fin d'injection. Pour les seringues jetables le retour à l'état initial est impossible. Les seringues devant. être utilisées plusieurs fois comportent des dispositifs auxiliaires pour recomprimer le ressort et remplir le réservoir de principe actif liquide ou le changer.

De même la demande de brevet WO 97/13537 décrit une seringue sans aiguille comportant une paroi déplaçable et un moyen de poussée constitué par une tige avec une tête de piston. Le moyen de poussée se met en mouvement quand la force résultante de la pression de gaz du générateur sur la tête du piston est supérieure à la force résultante des frottements du piston : la retenue temporaire ainsi réalisée par cet équilibre des forces se traduit par une mise en mouvement assez lente de la paroi déplaçable.

Ces dispositifs présentent plusieurs inconvénients. La biodisponibilité obtenue par ces dispositifs n'est pas entièrement satisfaisante. Rappelons que la biodisponibilité se définit par la quantité de liquide effectivement injecté par rapport à celle remplissant initialement le réservoir de la seringue.

Ces dispositifs sont encombrants et lourds car il faut prévoir la place pour réaliser une course assez importante pour l'accélération de la tige d'impact. De plus des moteurs à ressort ou gaz comprimés prévus pour stocker longuement, avant utilisation, un niveau d'énergie élevé vont être structurellement assez lourds. De plus ces dispositifs présentent des problèmes de fiabilité. Lors d'un stockage prolongé : le ressort comprimé au maximum se dégrade ; la réserve de gaz comprimé sera sujette à des fuites et le verrou fortement précontraint peut lui aussi présenter des difficultés de fonctionnement. Dans tous les cas ces dispositifs seront surdimensionnés pour tenter de pallier aux problèmes de fiabilité évoqués et donc seront encore un peu plus encombrant et un peu plus lourd.

L'objet de l'invention a pour but d'augmenter la biodisponibilité du principe actif en améliorant la phase de mise en vitesse du liquide et aussi de proposer des dispositifs plus compacts et fiables.

La présente invention concerne une seringue sans aiguille pour l'injection d'un principe actif liquide initialement placé entre d'une part un injecteur comportant au moins un conduit d'injection, ledit injecteur étant placé en contact ou à proximité immédiate de la peau du sujet à traiter et d'autre part une paroi déplaçable initialement séparée d'un moyen de poussée déplacé par les gaz d'un générateur de gaz et assurant la mise en pression et l'expulsion du principe actif liquide au travers de l'injecteur placé à l'extrémité aval de le seringue, ladite seringue étant telle que ledit moyen de poussée comporte un dispositif de retenue temporaire désactivé par le fonctionnement du générateur de gaz initié par un organe de déclenchement.

Initialement le moyen de poussée est séparé de la paroi déplaçable par une distance réduite dont nous expliciterons la détermination par la suite. Le fonctionnement du générateur de gaz désactive le moyen de retenue temporaire, déplace brutalement le moyen de poussée pour l'amener au contact de la paroi déplaçable assurant ainsi une mise en pression très rapide du liquide et son injection à vitesse élevée. Plus précisément l'opérateur agit sur un organe de déclenchement du fonctionnement du générateur de gaz, ces derniers agissant sur le moyen de poussée et ce faisant désactivent le moyen de retenue temporaire dudit moyen de poussée. Au contraire, dans les dispositifs de l'état de la technique, l'opérateur agit (directement ou non) sur un verrou, qui est un dispositif de retenue, pour libérer l'énergie nécessaire au fonctionnement du dispositif.

Dans cette invention par principe actif liquide nous entendrons essentiellement un liquide plus ou moins visqueux, ou un mélange de liquides, ou un gel. Le principe actif pourra être solide sous forme pulvérulente mis en suspension, plus ou moins concentrée, dans un liquide approprié. La granulométrie du principe actif solide et pulvérulent doit être adaptée, ainsi que la forme du conduit, pour éviter les bouchages des conduits.

Avantageusement pour cette seringue le dispositif de retenue temporaire sera frangible : il sera cassé lors du fonctionnement du générateur de gaz. Ledit système de retenue temporaire sera calibré c'est à dire que la rupture du dispositif de retenue temporaire frangible interviendra seulement quand le moyen de poussée, sous l'effet des gaz du générateur, sera soumis à une force donnée, dépendant notamment du principe actif et des conditions d'utilisations, pour obtenir très rapidement un jet de liquide de vitesse élevée.

Préférentiellement dans cette seringue la distance initiale séparant le moyen de poussée de la paroi déplaçable sera supérieure à la déformation maximale, avant rupture, du dispositif de retenue temporaire. En respectant cette condition, le moyen de poussée ne vient pas en contact avec la paroi déplaçable lors de la déformation du dispositif de retenue temporaire : il n'y a donc pas de liquide refoulé à très faible vitesse pendant la déformation du dispositif de retenue temporaire frangible.

Avantageusement la distance initiale séparant le moyen de poussée de la paroi déplaçable reste petite pour limiter l'encombrement de la seringue. Ladite distance est au plus de l'ordre de grandeur d'une dimension remarquable du moyen de poussée qui détermine la force agissant sur le dispositif de retenue temporaire frangible. Par exemple ladite distance restera inférieure à environ un dixième du diamètre ou du diamètre équivalent du moyen de poussée.

Préférentiellement le générateur de gaz motorisant la seringue est un générateur de gaz pyrotechnique. Ce type de générateur de gaz comprend, avant fonctionnement, un chargement pyrotechnique sous forme solide ou éventuellement pulvérulente, un dispositif pour initier la combustion dudit chargement et un organe de déclenchement dudit dispositif d'initiation. Contrairement au cas d'un ressort comprimé l'organe de déclenchement n'est soumis à aucune précontrainte.

Avantageusement le dispositif de retenue temporaire est choisi dans le groupe comprenant les goupilles : des goupilles cisaillables simples ou avec des zones d'affaiblissement, des ergots frangibles ; les opercules cisaillables qui seront cisaillées selon un cercle si le moyen de poussée est circulaire, des collerettes cisaillables.

Avantageusement le dispositif de retenue temporaire peut être aussi une tige axiale dont une extrémité est fixée au moyen de poussée et l'autre extrémité à un dispositif approprié du côté du générateur de gaz. La rupture de cette tige axiale intervient par allongement ; cette rupture peut être maîtrisée par une zone convenablement affaiblie de la tige.

On pourra éventuellement utiliser pour remplir la fonction de dispositif de retenue temporaire frangible des dispositifs de retenue se désactivant par déformation ou déplacement de certains éléments ; par exemples des dispositifs avec des crans qui seront déformés ou avec des cliquets qui seront déplacés quand lesdits dispositifs sont soumis à un effort important et prédéterminé appliqué au moyen de poussée.

Avantageusement encore le moyen de poussée sera un piston, pour limiter l'encombrement du dispositif. Ce piston comporte des dispositifs d'étanchéité par exemple un joint torique pour assurer son bon fonctionnement et éviter des fuites de gaz, vers le réservoir de principe actif.

Dans une première réalisation la paroi déplaçable sera aussi un piston, un piston de refoulement, avec des dispositifs d'étanchéité pour faire un deuxième niveau d'étanchéité. Le choix de matériaux appropriés pour le moyen de poussée et la paroi déplaçable permet de régler en quelque sorte, le choc lors de l'impact des deux pièces.

Dans cette réalisation les pistons constituants le moyen de poussée et le piston de refoulement ont des diamètres égaux ou différents. Toutefois lorsque le moyen de poussée a un diamètre supérieur à celui du piston de refoulement, ledit moyen de poussée a, à sa partie aval, une partie protubérante dont le diamètre est au plus égal à celui du piston de refoulement, la longueur de cette protubérance étant supérieure à la course du moyen de poussée.

Dans une deuxième réalisation la paroi déplaçable sera une membrane déformable. Cette membrane, de préférence mince, sera en métal ou en élastomère ou en matière plastique ; ces matériaux seront compatibles avec le principe actif. La fixation de ladite membrane dans la seringue assurera l'étanchéité. Cette fixation se fera, par exemple par pincement, sertissage ou surmoulage.

Dans cette réalisation, la face aval du moyen de poussée a une forme adaptée pour emboutir et déformer la membrane déformable sur la face intérieure de l'injecteur de façon, qu'en fin d'injection, la membrane déformée soit prise en « sandwich » entre la face aval du moyen de poussée et la face intérieure de l'injecteur, pour vider totalement le réservoir de principe actif. Nous dirons que la face aval du moyen de poussée et la face intérieure de l'injecteur ont des formes conjuguées.

Du fait de la puissance disponible avec un générateur de gaz pyrotechnique et du dispositif retenue temporaire frangible on obtient très rapidement un jet à grande vitesse pour perforer la peau, on améliore donc la biodisponibilité du principe actif.

L'encombrement et le poids de la seringue sont réduits par la limitation du déplacement du moyen de poussée vers la paroi déplaçable. La compacité d'un générateur de gaz pyrotechnique ajoute encore à cet effet.

Enfin la fiabilité des générateurs de gaz pyrotechnique est excellente, notamment pendant toute la phase qui va de l'assemblage jusqu'à l'utilisation aucun élément du générateur de gaz pyrotechnique n'est soumis à une précontrainte.

Un autre avantage de cette seringue est lié à la séparation du moyen de poussée et de la paroi déplaçable : on peut distinguer deux sous-ensembles dans la seringue. Le premier contient le principe actif il est préparé et conditionné dans les conditions inhérentes à l'industrie pharmaceutique, l'autre contient le générateur de gaz, de même il est préparé et conditionné dans les conditions inhérentes à ce type d'appareil. Les deux sous-ensembles étant assemblés dans un atelier dans lequel des contraintes réglementaires et techniques sont très limitées.

Ci-dessous l'invention est exposée plus en détail à l'aide de figures représentant des réalisations particulières.

La figure 1 représente, schématiquement et en coupe partielle, une première réalisation de la seringue avec un piston de poussée et un piston de refoulement .

La figure 2 représente, en coupe partielle, une deuxième réalisation avec une membrane mince déformable.

La figure 3 représente le détail de la partie aval de la seringue précédente après utilisation.

Pour faciliter les descriptions les seringues seront supposées verticales avec leur partie aval dirigée vers le bas.

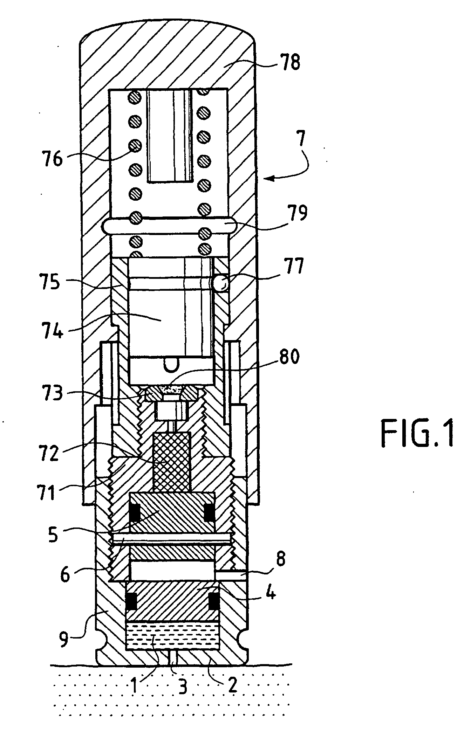

La figure 1 représente, en coupe longitudinale partielle, une seringue sans aiguille selon l'invention. La partie aval 9 de la seringue comporte un injecteur 2 avec un seul conduit d'injection 3 dans cet exemple. L'injecteur 2 est en appui sur la peau du sujet à traiter. Dans le réservoir, formé par la partie intérieure de la partie aval 9 de la seringue fermée par le piston de refoulement 4, se trouve le principe actif liquide 1. Le piston de refoulement 4 comporte un joint torique pour assurer l'étanchéité. Dans le corps 71, fixé par vissage sur la partie aval 9, se trouve le moyen de poussée ici un piston 5, avec un joint torique pour assurer l'étanchéité. Ledit piston 5 est maintenu temporairement en place par une goupille 6 traversant le piston et le corps 71. Dans cet exemple le piston de poussée 5 a un diamètre légèrement inférieur à celui du piston de refoulement 4, le piston de poussée ne nécessite pas d'aménagement particulier comme nous l'avons décrit précédemment pour assurer un fonctionnement correct. Le piston de poussée 5 est placé à une petite distance du piston de refoulement 4. Cette distance est supérieure à la déformation maximale de la goupille avant rupture. Le piston 5 lors de cette déformation lente ne vient pas au contact du piston 4 et il n'y a donc pas de liquide refoulé à faible vitesse. Le piston 5 est à une distance égale à environ deux fois le diamètre de la goupille, pour limiter cette distance et réduire l'encombrement.

Le volume compris entre le piston de poussée 5 et le piston de refoulement 4 communique éventuellement avec l'extérieur par au moins un évent, tel que l'évent 8, cet évent est percé à travers la partie aval 9 et le corps 71 et sert à évacuer l'air compris entre les deux pistons lors du fonctionnement.

Le piston 5 est, dans cet exemple, déplacé par un générateur de gaz pyrotechnique 7 dont nous allons décrire les principaux éléments. Le générateur de gaz pyrotechnique 7 comprend dans le corps 71, au dessus du piston de refoulement 5, un chargement pyrotechnique 72 dont la combustion est initiée par une amorce 80 impactée par un percuteur 74, ce percuteur n'est pas représenté en coupe mais vu de côté. L'amorce 80 est logée dans un porte-amorce 73. En position initiale le percuteur 74 est retenu, dans le guide-percuteur 75 solidaire par vissage du corps 71, par au moins une bille, telle que la bille 77, partiellement engagée dans une gorge du percuteur. Le dispositif de percussion comprend un poussoir 78 avec une gorge 79 et un ressort intérieur 76.

Le poussoir 78 coulisse sur l'extérieur du guide-percuteur 75 et il est retenu pas des ergots se déplaçant dans des rainures latérales. Ce poussoir 78 est ici l'organe de déclenchement.

Bien entendu pour initier la combustion du chargement pyrotechnique 72, sans sortir du cadre de l'invention, on peut utiliser des dispositifs d'initiation autres que le dispositif à percuteur ici décrit. Sans entrer dans les détails et sans vouloir être exhaustif, nous citerons comme exemples des dispositifs d'initiation à pile électrique ou des dispositifs d'initiation piézo-électrique.

Eventuellement le générateur de gaz pyrotechnique peut être remplacé par un générateur de gaz constitué par un réservoir de gaz comprimé fermé par une vanne à ouverture rapide. L'organe de déclenchement va ouvrir ladite vanne, les gaz comprimés du réservoir vont se détendre et agir sur le moyen de poussée pour désactiver le dispositif de retenue temporaire, pour accélérer, le moyen de poussée et pour le déplacer avec la paroi déplaçable pour faire l'injection.

Sur cette figure 1 la seringue est prête à l'emploi, en appui sur la peau du sujet à traiter. L'opérateur appui, avec son pouce, sur le poussoir 78 qui s'enfonce en comprimant le ressort 76. Le poussoir se déplace jusqu'à ce que la gorge 79 arrive à la hauteur de la gorge du percuteur 74, les billes, telle que la bille 77, retenant le percuteur 74, se dégagent dans la gorge 79 et libèrent le percuteur qui va impacter violemment l'amorce 80, dont l'initiation enflamme le chargement pyrotechnique 72. Le percuteur en appui sur le porte-amorce 73 assure l'étanchéité : les gaz de combustion ne remontant pas vers le poussoir.

La combustion du chargement pyrotechnique va produire des gaz qui agissent sur le piston de poussée 5. La pression au dessus de ce piston augmente jusqu'à ce que la force résultante soit suffisante pour cisailler la goupille 6. Lorsque la goupille 6 casse, le piston de poussée 5 est très rapidement accéléré car soumis à une force importante. Le piston de poussée 5 impacte le piston de refoulement 4 et le déplace très rapidement. Le principe actif liquide 1 est éjecté par le conduit 3 de l'injecteur 2 à une vitesse élevée : il perce facilement la peau et diffuse plus ou moins profondément à travers elle. La cinétique de combustion du chargement pyrotechnique 72 et le calibrage de la goupille 6 permettent de régler la vitesse du jet liquide.

Dans une réalisation plus élaborée la goupille, fabriquée dans un matériau plus résistant au cisaillement, comporte deux sections affaiblies au droit de la surface extérieure du piston de poussée. Les cassures de la goupille seront de sections plus petites et perturberont moins le fonctionnement du piston de poussée. Les entailles, correspondant aux zones de sections affaiblies, sont dimensionnées pour que la rupture intervienne pour la même pression qu'avec une goupille simple, fabriquée dans un matériau moins résistant.

Pour le système de retenue temporaire et frangible, le piston de poussée, le corps 71 et la goupille 6 seront préférentiellement métalliques, par exemple en aciers convenablement choisis. Le piston de refoulement 4, en contact avec le principe actif liquide sera fabriqué dans un matériau métallique ou plastique ou élastomère compatible avec ledit principe actif.

Le chargement pyrotechnique est constitué, par exemple, par une poudre à base de nitrocellulose, poudre dont la granulométrie est choisie pour donner une cinétique de combustion appropriée, par exemple 120mg de poudre BTu suivant le catalogue de SNPE qui permet de cisailler une goupille en acier dur de 1,5mm de diamètre pour une pression de 9 Mpa sur un piston de 12mm de diamètre.

On peut aussi commenter sur cette figure 1 l'avantage que procure l'utilisation d'un piston de poussée 5 séparé d'un piston de refoulement 4 ou, plus généralement d'un moyen de poussée séparé d'une paroi déplaçable. La seringue peut être séparée en deux sous-ensembles. Le premier sous-ensemble comprendra la partie aval 9 de la seringue contenant le principe actif liquide 1 fermée par le piston de refoulement 4 : ce sous-ensemble peut être assemblé et rempli dans un atelier répondant aux normes de fabrication pharmaceutique notamment au point de vue asepsie ; l'asepsie de la face aval de l'injecteur 2 devra être protégée par un bouchon approprié qui évitera aussi des pertes de liquide au cours des manipulations qui suivent le remplissage. Le second sous-ensemble relève d'une préparation et d'un assemblage dans un atelier répondant aux normes de la pyrotechnie, ce sous-ensemble sera convenablement sécurisé pour empêcher des déplacements du poussoir qui seraient à l'origine de fonctionnements intempestifs du générateur de gaz. Ses deux sous-ensembles préparés séparément seront enfin assemblés dans une environnement nécessitant moins de précautions, pour réaliser la seringue complète ; elle sera ensuite conditionnée pour livraison aux clients.

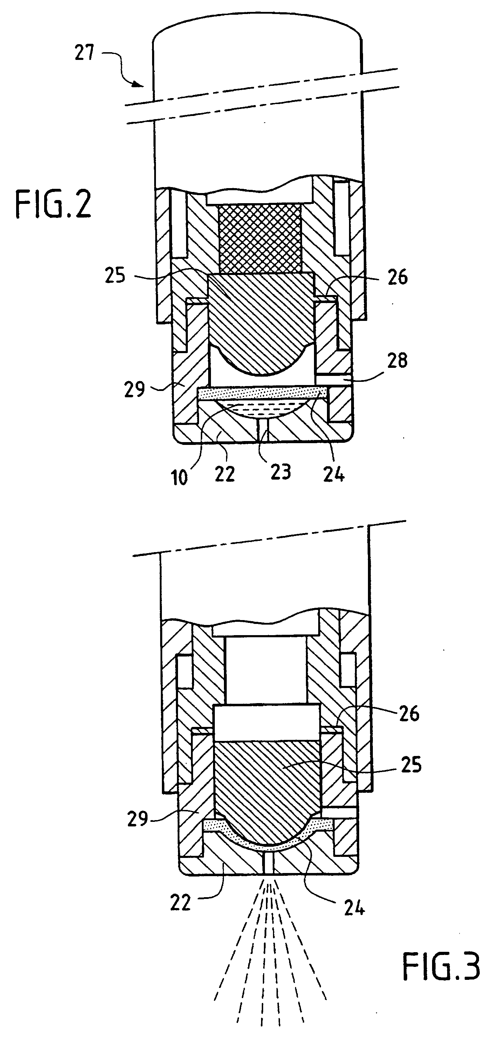

La figure 2 décrit une autre réalisation d'une seringue selon l'invention. Elle diffère de la précédente notamment par la forme du piston de poussée, par le dispositif de retenue temporaire et la paroi déplaçable.

On trouve sur cette figure un injecteur 22 avec un conduit d'injection 23. La partie interne de l'injecteur a sensiblement la forme d'une calotte sphérique, elle contient le principe actif liquide 10, une membrane mince déformable 24 ferme le réservoir. Dans cet exemple: la membrane mince est pincée entre l'injecteur 22 et la pièce de guidage 29. La membrane mince déformable 24 peut aussi être sertie ou surmoulée sur l'injecteur 22. Au dessus de cette membrane déformable se trouve le piston de poussée 25 maintenu par une collerette frangible 26 pincée entre la pièce de guidage 29 et le corps du générateur de gaz. Le piston de poussée 25 a sur sa face aval, vers la membrane mince déformable 24, une forme de calotte sphérique conjuguée de celle de l'injecteur. La distance séparant le piston de poussée 25 de la membrane mince déformable est inférieure à deux fois l'épaisseur de la collerette frangible 26. Dans cette figure les épaisseurs de la membrane mince déformable 24, de la collerette frangible 26 ont été exagérées pour la lisibilité du dessin.

Dans cet exemple les formes conjuguées de la face avant du piston de poussée et la face interne de l'injecteur sont des calottes sphériques, des formes plus élaborées sont aussi envisageables.

Le piston de poussée est déplacé par un générateur de gaz pyrotechnique, repéré par 27, ce générateur est identique à celui de l'exemple, sa description ne sera pas reprise en détail.

Lorsque la combustion du chargement pyrotechnique est initiée, les gaz produits font monter la pression sur la face amont du piston de poussée 25 jusqu'à ce la force résultante cisaille la collerette, le piston de poussée 25 est très rapidement accéléré car soumis à une force importante. Le piston de poussée 25 impacte la membrane déformable 24 et l'embouti contre la face intérieure de l'injecteur 22, ce faisant le principe actif est éjecté, par le conduit 23 de l'injecteur 22, à une vitesse élevée comme dans l'exemple précédent.

La figure 3 représente schématiquement la partie aval de la seringue de l'exemple précédent en fin de fonctionnement. Le chargement pyrotechnique a entièrement brûlé. La collerette 26 du piston a été cisaillée suivant une circonférence, elle reste entre les pièces 29 et 71. Le piston de poussée 25 a embouti et déformé la membrane déformable 24.

## Revendications

1. Seringue sans aiguille motorisée par un générateur de gaz (7,27) pour l'injection d'un principe actif liquide (1,10) contenu dans un réservoir à l'intérieur de la partie aval (9,29) de ladite seringue, ledit réservoir comprend du côté aval un injecteur (2,22) avec au moins un conduit d'injection (3,23) et du côté opposé une paroi déplaçable (4,24) ladite paroi déplaçable étant distante d'un moyen de poussée (5,25) comportant un dispositif de retenue temporaire calibré **caractérisé en ce que** ledit dispositif de retenue temporaire est un dispositif frangible (6,26).

2. Seringue sans aiguille selon la revendication 1 **caractérisée en ce que** la distance initiale séparant le moyen de poussée (5,25) de la paroi déplaçable (4,24) est supérieure à la déformation maximale avant rupture du dispositif de retenue temporaire frangible (6,26).

3. Seringue sans aiguille selon la revendication 1 ou 2 **caractérisée en ce que** la distance initiale séparant le moyen de poussée (5,25) de la paroi déplaçable (4,24) est inférieure à environ un dixième du diamètre du moyen de poussée.

4. Seringue sans aiguille selon l'une des revendications précédentes **caractérisée en ce que** le générateur de gaz (7,27) est un générateur de gaz pyrotechnique.

5. Seringue sans aiguille selon l'une des revendications 1 à 4 **caractérisée en ce que** le dispositif de retenue temporaire (6,26) est choisi dans le groupe comprenant notamment les goupilles et les opercules.

6. Seringue sans aiguille selon l'une des revendications 1 à 3 **caractérisée en ce que** le moyen de poussée (5,25) est un piston.

7. Seringue sans aiguille selon l'une des revendications 1 à 6 **caractérisée en ce que** la paroi déplaçable (4) est un piston de refoulement.

8. Seringue sans aiguille selon l'une des revendications 1 à 6 **caractérisée en ce que** la paroi déplaçable (24) est une membrane déformable.

9. Seringue sans aiguille selon la revendication 8 **caractérisée en ce que** la face aval du moyen de poussée (25) et la face intérieure de l'injecteur (22) ont des formes conjuguées.

## Patentansprüche

1. Nadellose Spritze, die von einem Gasgenerator (7, 27) angetrieben wird, zum Einspritzen eines flüssigen Wirkstoffs (1, 10), der in einem Behälter innerhalb des stromabwärtsliegenden Bereichs (9, 29) der Spritze enthalten ist, wobei der Behälter auf der stromabwärts liegenden Seite eine Einspritzdüse (2, 22) mit mindestens einem Einspritzkanal (3, 23) und auf der entgegengesetzten Seite eine verschiebbare Wand (4, 24) aufweist, wobei die verschiebbare Wand in Abstand zu einem Schubmittel (5, 25) angeordnet ist, das eine kalibrierte Vorrichtung zur vorübergehenden Zurückhaltung aufweist, **dadurch gekennzeichnet, dass** die Vorrichtung zur vorübergehenden Zurückhaltung eine zerreißbare Vorrichtung (6, 26) ist.

2. Nadellose Spritze nach Anspruch 1, **dadurch gekennzeichnet, dass** der das Schubmittel (5, 25) von der verschiebbaren Wand (4, 24) trennende, ursprüngliche Abstand größer ist als die maximale Verformung der zerreißbaren Vorrichtung (6, 26) vor dem Zerreißen.

3. Nadellose Spritze nach Anspruch 1 oder 2, **dadurch gekennzeichnet, dass** der das Schubmittel (5, 25) von der verschiebbaren Wand (4, 24) trennende, ursprüngliche Abstand kleiner als etwa ein Zehntel des Durchmessers des Schubmittels ist.

4. Nadellose Spritze nach einem der vorhergehenden Ansprüche, **dadurch gekennzeichnet, dass** der Gasgenerator (7, 27) ein pyrotechnischer Gasgenerator ist.

5. Nadellose Spritze nach einem der Ansprüche 1 bis 4, **dadurch gekennzeichnet, dass** die Vorrichtung zur vorübergehenden Zurückhaltung (6, 26) aus der Gruppe ausgewählt wird, zu der insbesondere die Stifte und die Abdeckfolien gehören.

6. Nadellose Spritze nach einem der Ansprüche 1 bis 3, **dadurch gekennzeichnet, dass** das Schubmittel (5, 25) ein Kolben ist.

7. Nadellose Spritze nach einem der Ansprüche 1 bis 6, **dadurch gekennzeichnet, dass** die verschiebbare Wand (4) ein Druckkolben ist.

8. Nadellose Spritze nach einem der Ansprüche 1 bis 6, **dadurch gekennzeichnet, dass** die verschiebbare Wand (24) eine verformbare Membran ist.

9. Nadellose Spritze nach Anspruch 8, **dadurch gekennzeichnet, dass** die stromabwärts liegende Seite des Schubmittels (25) und die Innenseite der Einspritzdüse (22) einander zugeordnete Formen haben.

## Claims

1. A needleless syringe driven by a gas generator (7, 27) for injection of a liquid active principle (1, 10) contained in a reservoir inside the downstream part (9, 29) of said syringe, said reservoir having, at the downstream end, an injector (2, 22) with at least one injection conduit (3, 23) and, at the opposite end, a displaceable wall (4, 24), said displaceable wall being separated from a thrust means (5, 25) which has a calibrated temporary retention device, **characterized in that** said temporary retention device is a breakable device (6, 26).

2. The needleless syringe as claimed in claim 1, **characterized in that** the initial distance separating the thrust means (5, 25) from the displaceable wall (4, 24) is greater than the maximum deformation before rupture of the breakable temporary retention device (6, 26).

3. The needleless syringe as claimed in claim 1 or 2, **characterized in that** the initial distance separating the thrust means (5, 25) from the displaceable wall (4, 24) is less than about one tenth of the diameter of the thrust means.

4. The needleless syringe as claimed in one of the preceding claims, **characterized in that** the gas generator (7, 27) is a pyrotechnic gas generator.

5. The needleless syringe as claimed in one of claims 1 through 4, **characterized in that** the temporary retention device (6, 26) is chosen from the group including in particular pins and protective caps.

6. The needleless syringe as claimed in one of claims 1 through 3, **characterized in that** the thrust means (5, 25) is a piston.

7. The needleless syringe as claimed in one of claims 1 through 6, **characterized in that** the displaceable wall (4) is a delivery piston.

8. The needleless syringe as claimed in one of claims 1 through 6, **characterized in that** the displaceable wall (24) is a deformable membrane.

9. The needleless syringe as claimed in claim 8, **characterized in that** the downstream face of the thrust means (25) and the inner face of the injector (22) have matching shapes.
